# EUROPÄISCHE PATENTSCHRIFT

(11) **EP 0 447 862 B1**
(45) Veröffentlichungstag und Bekanntmachung des Hinweises auf die Patenterteilung: **12.01.1994**
(21) Anmeldenummer: 91103147.4
(22) Anmeldetag: 02.03.1991
(51) Int. Cl.: B01D 53/00, C12N 1/36, C12S 5/00, C12N 1/26, A62D 3/00

(54) **Verfahren zur mikrobiologischen Reinigung von mit halogenierten Ethenen und/oder mit halogenierten Butadienen kontaminierten Gasströmen**
Method for the biological purification of gases which are contaminated with halogenated ethenes and/or with halogenated butadienes
Méthode pour la purification biologique de gaz contaminés par des éthènes halogénés et/ou par des butadiènes halogénés

(30) Priorität: 21.03.1990 DE 4009109
(43) Veröffentlichungstag der Anmeldung: 25.09.1991
(73) Patentinhaber: FRAUNHOFER-GESELLSCHAFT ZUR FÖRDERUNG DER ANGEWANDTEN FORSCHUNG E.V., 80636 München (DE)
(72) Erfinder: Knackmuss, Hans-Joachim, Prof. Dr., W-7250 Leonberg 5 (DE); Ewers, Jens, Dipl.-Chem., W-7000 Stuttgart 40 (DE); Clemens, Wulf-Christian, Dipl.-Ing., W-7000 Stuttgart 1 (DE)

(56) Entgegenhaltungen:
- EP-A- 0 328 708
- EP-A- 0 336 718
- DE-A- 3 227 678
- GB-A- 2 067 092

## Beschreibung

Die Erfindung betrifft ein Verfahren zur mikrobiologischen Reinigung von mit halogenierten Ethenen und/oder mit halogenierten Butadienen kontaminierten Gasströmen.

Halogenierte Ethene und halogenierte Butadiene besitzen ein breites Anwendungsspektrum. So werden z.B. chlorierte Ethene in den unterschiedlichsten Bereichen als Lösungs- und/oder als Reinigungsmittel eingesetzt. Kontaminationen von Böden, Wasser und Abluft mit diesen meist flüchtigen Substanzen treten deshalb sehr häufig auf und führen zu erheblichen Umweltbelastungen.

Die Sanierung belasteter Standorte geschieht vorwiegend durch Boden-Luft-Absaugung. Dabei gehen die flüchtigen Komponenten, z.B. chlorierte Ethene, in den Abluftstrom über, welcher anschließend über Aktivkohlefilter geleitet wird, sodaß die Verunreinigungen, z.B. die chlorierten Ethene, durch Adsorption aus dem Abluftstrom entfernt werden. Anschließend wird die Aktivkohle regeneriert und erneut zur Reinigung der Abluftströme eingesetzt. Falls eine Regenerierung der Aktivkohle nicht wirtschaftlich ist, wird diese zusammen mit dem Adsorbat verbrannt. Der Einsatz sowie die Regenerierung von Aktivkohle ist meist dann unwirtschaftlich, wenn in dem zu reinigenden Abluftstrom nur geringe Konzentrationen an den zu entfernenden Verunreinigungen vorhanden sind, so daß die Aktivkohle gemäß Adsorptionsisotherme nur gering beladen wird und deshalb zur Reinigung der Abluftströme große Mengen an Aktivkohle erforderlich sind. In der Praxis werden Abluftströme, die nur geringe Konzentrationen an Verunreinigungen enthalten, ungereinigt in die Umwelt abgegeben. Andererseits ist man aber aus umweltpolitischen Gründen bestrebt, auch diese Gasströme zu reinigen und zu entsorgen. So wird z.B. Trichlorethylen aus Grundwasserleitern dadurch abgehalten, daß Luft durch das Wasser geleitet wird, damit das Trichlorethylen in die Abluft übergeht. Die Konzentrationen dieser Gasströme an Trichlorethylen liegen dabei nach einer kurzen Anlaufphase mit hohen Trichlorethylen-Konzentrationen unterhalb der Grenzwerte der TA-Luft. Aufgrund der heutigen gesetzlichen Vorschriften kann deshalb diese Abluft ohne weitere Reinigung in die Atmosphäre abgegeben werden, sodaß letztendlich nur eine Umverteilung des Trichlorethylens stattfindet. Man ist aber bestrebt, durch diese Art der Grundwasserreinigung nicht nur eine Umverteilung des Trichlorethylens zu bewirken, sondern dieses auch dauerhaft zu entsorgen. Will man Gasströme mit diesen geringen Konzentrationen an Trichlorethylen über Aktivkohle reinigen, so ist dies sehr kostenintensiv, da bei diesen geringen Konzentrationen die Aktivkohle nur gering beladen wird und für eine Reinigung dieser Gasströme riesige Mengen an Aktivkohle erforderlich wären. Eine Regenerierung der Aktivkohle ist ebenfalls nicht wirtschaftlich, sodaß die Entsorgung durch Verbrennen oder Deponierung der Aktivkohle erfolgt.

Eine andere Möglichkeit, um z.B. mit Trichlorethylen kontaminierte Böden zu reinigen, besteht darin, Methan in das Erdreich zu injizieren, um Methan oxidierende Bakterien, die mittels einer Monooxygenase Trichlorethylen epoxidieren können, anzureichern. Der mikrobiologische Abbau von chlorierten Ethenen, z. B. von Trichlorethylen, durch Methan und Aromaten verwertende Bakterien erfolgt durch den Initialangriff einer Oxygenase auf das chlorierte Ethen und führt zu einem sehr reaktiven Epoxid. Dieses Epoxid alkyliert Proteine und schädigt bzw. desaktiviert somit die Zellen. Wird z.B. als Mikroorganismus ein Pseudomonas putida F1 Stamm verwendet, so verringert sich dessen Aktivität für den Abbau von Trichlorethylen nach 20 Minuten um 98 %.

In der EP-A-0 336 718 wird ein Verfahren beschrieben, mit dem mittels genetisch manipulierter Mikroorganismen Trichlorethylen mikrobiologisch abgebaut wird. Da es sich hierbei um genetisch manipulierte Mikroorganismen handelt, sind vor dessen Einsatz umfangreiche und langwierige Genehmigungsverfahren notwendig. Ferner sind genetische Manipulationen an Mikroorganismen technisch sehr aufwendig, deshalb sehr teuer und somit in vielen Fällen unwirtschaftlich. Zu Vergleichszwecken werden auch natürlich vorkommende, TCE abbauende Bakterienstämme beschrieben. Eine präadaptierung von Alkenverwertern mit Isopren u/o Butadienen wird nicht beschrieben.

Aus der DE-A-3326057 ist ein Verfahren zur biologischen Abluftreinigung bekannt, es wird ganz allgemein ein Biowäscher beschrieben. Die dort eingesetzte Biologie ist allerdings nur in der Lage, einfach und zweifach chlorierte Alkane sowie Chlorbenzol und Chlortoluol abzubauen, nicht jedoch chlorsubstituierte Alkene wie z.B. Trichlorethen zu mineralisieren.

In der DE-A-3227678 ist ein Verfahren zur biologischen Reinigung von Abluft beschrieben. Hierbei handelt es sich ganz allgemein um einen Biofilter, und die eingesetzte Biologie ist auch hier nicht in der Lage, halogenierte Alkene zu mineralisieren.

Der Erfindung liegt deshalb die Aufgabe zugrunde, ein Verfahren bereitzustellen, mit dem Gasströme, die mit halogenierten Ethenen und/oder mit halogenierten Butadienen kontaminiert sind, auf wirtschaftliche Weise, schnell und einfach gereinigt werden können. Diese Kontaminationen sollen nicht nur aus den Gasströmen entfernt werden, sondern sie sollen auch auf einfache und wirtschaftliche Weise vernichtet werden. Ferner soll das Verfahren in der Lage sein, auch solche Gasströme wirtschaftlich reinigen und entsorgen zu können, deren Konzentrationen an halogenierten Ethenen und/oder an halogenierten Butadienen sehr gering sind und z.B. unterhalb der Grenzwerte der TA Luft liegen.

Gelöst wird diese Aufgabe dadurch, daß man geeignete Bakterienstämme, welche mit Isopren und/oder mit Butadienen als Kohlenstoff- und Energiequelle wachsen und welche halogenierte Ethene und/oder halogenierte Butadiene abbauen, präadaptiert, in dem man sie in einem Bioreaktor mit Isopren und/oder mit Butadienen als Kohlenstoff- und Energiequelle für die Neubildung oder für die Regenerierung versorgt, daß man den zu reinigenden Gasstrom in einen Bioreaktor leitet, welcher die präadaptierten Bakterien enthält, und daß in diesem Bioreaktor die halogenierten Ethene und die halogenierten Butadiene aus dem Gasstrom durch biologischen Abbau mittels der präadaptierten Bakterien entfernt werden. Geeignete Butadiene zur Durchführung des erfindungsgemäßen Verfahrens sind z.B. 1,2-Butadien und/oder 1,3-Butadien.

Mit Hilfe des erfindungsgemäßen Verfahrens ist es möglich geworden, auch solche Gasströme wirtschaftlich zu reinigen und zu entsorgen, deren Konzentrationen an halogenierten Ethenen und/oder an halogenierten Butadienen sehr gering sind und z.B. unterhalb der Grenzwerte der TA-Luft liegen. Mit Hilfe des erfindungsgemäßen Verfahrens ist es möglich geworden, auch solche Gasströme wirtschaftlich reinigen und entsorgen zu können, deren Konzentrationen an halogenierten Ethenen und/oder an halogenierten Butadienen im Bereich von 1 - 100 mg/m³ Luft liegen.

Mit Hilfe des erfindungsgemäßen Verfahrens ist es ferner möglich geworden, auch mit halogenierten Ethenen und/oder mit halogenierten Butadienen kontaminierte Böden oder Abwässer zu reinigen.

Die Reinigung dieser kontaminierten Abwässer erfolgt in einem ersten Verfahrensschritt durch sog. "Strippen" des Abwassers. Dabei wird Luft in das zu reinigende Abwasser eingeblasen, die flüchtigen Kontaminationen treten in den Abluftstrom, und der kontaminierte Abluftstrom wird, wie oben beschrieben, nach dem erfindungsgemäßen Verfahren gereinigt.

Die Reinigung der kontaminierten Böden kann auf drei verschiedene Arten erfolgen. In einer ersten Ausführungsform wird Luft in den zu reinigenden Boden eingeblasen, die flüchtigen Kontaminationen treten in den Abluftstrom, und der kontaminierte Abluftstrom wird, wie oben beschrieben, nach dem erfindungsgemäßen Verfahren gereinigt. In einer zweiten Ausführungsform wird die Bodenluft zusammen mit den flüchtigen Kontaminationen abgesaugt und, wie oben beschrieben, nach den erfindungsgemäßen Verfahren gereinigt. Dabei hängt es von der Durchlässigkeit des Bodens ab, ob die erste oder die zweite Ausführungsform zweckmäßiger ist. In einer dritten Ausführungsform wird der kontaminierte Boden einer Bodenwäsche unterzogen, dabei gehen die Kontaminationen in das Waschwasser, und das kontaminiert Waschwasser wird, wie oben für Abwässer beschrieben, nach dem erfindungsgemäßen Verfahren weiter behandelt.

Überraschender Weise wurde gefunden, daß Bakterien, die mit Isopren und/oder mit Butadien als Kohlenstoff- und Energiequelle wachsen, sehr hohe Konzentrationen an halogenierten Ethenen und/oder an halogenierten Butadienen, wie z.B. an Trichlorethylen, tolerieren können. Eine Zellschädigung der auf diese Weise präadaptierten Bakterien durch das entstehende Epoxid wird durch ein Epoxid entgiftendes Enzymsystem verhindert. Dieses Enzymsystem wird mit Isopren und/oder mit Butadienen als Kohlenstoff- und Energiequelle induziert. Die Bakterien können also durch das Wachstum mit z. B. Isopren als Strukturanaloga für den Abbau der halogenierten Ethene und/oder der halogenierten Butadienen präadaptiert werden. Bei auf diese Weise präadaptierten Bakterien wird selbst bei hohen Konzentrationen von z.B. Trichlorethylen eine Dehalogenierung beobachtet. So bleibt die Aktivität von präadaptierten Ruhezellen bei Abwesenheit geeigneter Kohlenstoff- und Energiequellen z.B. gegenüber Trichlorethylen mehrere Stunden erhalten und nimmt nur durch den allmählich auftretenden Energiemangel ab. Zur Reaktivierung der Ruhezellen für den Abbau von z.B. Trichlorethylen ist es deshalb erforderlich, diese mit Isopren und/oder mit Butadienen als Kohlenstoff- bzw. als Energiequelle zu regenerieren. Aus der folgenden Reaktionsgleichung ist ersichtlich, daß die präadaptierten Bakterien beim Abbau von z.B. Trichlorethylen keine Energie gewinnen können und deshalb bei nachlassender Aktivität für den weiteren Abbau von Trichlorethylen mit Energie versorgt werden müssen.

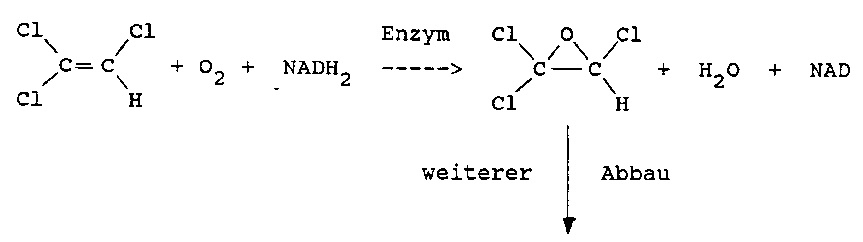

Besonders gute Resultate bei der Durchführung des erfindungsgemäßen Verfahrens erhält man, wenn es sich bei den zu entfernenden halogenierten Ethenen und/oder halogenierten Butadienen um chlorierte oder bromierte Ethene handelt, wie z.B. um Trichlorethylen, um cis- oder trans-1,2-Dichlorethen oder um Vinylchlorid. Mit Hilfe des erfindungsgemäßen Verfahrens können somit auch Altlasten beseitigt werden, die durch Kontamination mit Perchlorethylen entstanden sind, da Perchlorethylen biologisch unter methanogenen Bedingungen zu dem hochtoxischen und cancerogenen Vinylchlorid abgebaut wird, welches dann nach dem erfindungsgemäßen Verfahren weiter abgebaut werden kann.

Geeignete Bakterien zur Durchführung des erfindungsgemäßen Verfahrens erhält man, indem man aus Boden- oder Wasserproben nach bekannten Methoden diejenigen Bakterien mittels Isopren und/oder mittels Butadienen als einzigen Kohlenstoff- und Energiequellen isoliert, die in der Lage sind, Isopren und/oder Butadiene abzubauen. Mit Hilfe eines Schnelltestes werden dann aus diesen Bakterien diejenigen ermittelt, die halogenierte Ethene und/oder halogenierte Butadiene abbauen können. Die so ermittelten und isolierten Bakterien werden dann zur Durchführung des erfindungsgemäßen Verfahrens eingesetzt. Die Anzucht der präadaptierten Bakterien mit Isopren und/oder mit Butadienen kann in jedem beliebigen Fermenter erfolgen.

Überraschender Weise wurde festgestellt, daß auf den Strukturanaloga Isopren und Butadien wachsende Bakterien folgende charakteristische Eigenschaften besitzen können.
1. Eine hohe Toleranz gegenüber halogenierten Ethenen und halogenierten Butadienen, wie z. B. gegenüber Trichlorethylen.
2. Eine hohe Enzymaktivität für halogenierte Ethene und halogenierte Butadiene, wie z. B. für Trichlorethylen.
3. Eine enzymatische Detoxifikation von Zwischenprodukten.
4. Eine langanhaltende Abbauleistung bei Ruhezellen.

Das Enzymsystem für die Detoxifikation der Zwischenprodukte wird durch das Isopren und/oder durch die Butadiene als Kohlenstoff- und Energiequelle induziert. Geeignete Bakterien zur Durchführung des erfindungsgemäßen Verfahrens sind z.B. unter den Alkenverwertern zu finden. Ohne Einschränkung der Allgemeinheit sind geeignete Bakterien zur Durchführung des erfindungsgemäßen Verfahrens z.B. Alcaligenes denitrificans ssp. xylosoxidans Stämme oder Rhodococcus erythropolis Stämme.

Die Versorgung der Bakterien mit Isopren und/oder mit Butadienen als Kohlenstoff- und Energiequelle kann über die Gasphase erfolgen, oder durch Zugabe der entsprechenden Flüssigkeiten zu den Bakteriensuspensionen. Befinden sich die zuversorgenden Bakterien in einem Bioreaktor, so erfolgt zweckmäßigerweise die Zugabe des Isoprens und/oder der Butadiene über die Gasphase. Befinden sich die zu versorgenden Bakterien in einem geschlossenen System, z.B. in einem Schüttelkolben, so erfolgt die Zugabe, indem die entsprechenden Flüssigkeiten in den Kolben gegeben werden. Bevorzugte Ausführungsformen des erfindungsgemäßen Verfahrens verwenden Isopren oder 1,3-Butadien als Kohlenstoff- und Energiequelle. Bei weiteren bevorzugten Ausführungsformen des erfindungsgemäßen Verfahrens erfolgt die Versorgung der Bakterien mit Isopren oder mit 1,3-Butadien über die Gasphase, indem gasförmiges Isopren oder gasförmiges 1,3-Butadien dem Luftstrom für die Begasung des Bioreaktors zugemischt wird. Der Anteil z.B. des 1,3-Butadiens am Gasstrom ist dabei abhängig vom Volumenstrom, von der Strömungsgeschwindigkeit im Bioreaktor, von der Biomassenkonzentration im Bioreaktor, vom Nährstoffbedarf der Bakterien und vom Bioreaktortyp, und richtet sich nach den Anforderungen des jeweiligen Einzelfalles. Der Anteil von 1,3-Butadien am Gasstrom kann z.B. 10 000 ppm betragen, er kann aber auch höher oder niedriger sein. Die zum Wachstum bzw. zur Regenerierung der Bakterien erforderlichen Nährsalze werden in bekannter Weise in einem gepufferten System zugegeben.

Die Reinigung des Gasstromes erfolgt durch Einleiten des Gasstromes in den Bioreaktor, welcher die präadaptierten Bakterien für den Abbau enthält. Bevorzugte Ausführungsformen des erfindungsgemäßen Verfahrens arbeiten mit Blasensäulenreaktoren, mit Festbettreaktoren oder mit Membranreaktoren. Wird ein Blasensäulenreaktor zur Durchführung des erfindungsgemäßen Verfahren eingesetzt, so wird der zu reinigende Abluftstrom von unten durch die wässrige, die präadaptierten Bakterien enthaltende Biosuspension geleitet. Wird ein Festbettreaktor zur Durchführung des erfindungsgemäßen Verfahrens eingesetzt, so enthält dieser die präadaptierten Bakterien immobilisiert an Füllkörpern, z.B. an Siran^{R}-Ringen der Firma Schott. Der zu reinigende Gasstrom wird zusammen mit der wässrigen Phase für die Versorgung der präadaptierten Bakterien durch das Substrat geleitet. Wird ein Membranreaktor zur Durchführung des erfindungsgemäßen Verfahren eingesetzt, so wird der zu reinigende Gasstrom durch Hohlfasermembranen, die durch die wässrige, die präadaptierten Bakterien enthaltende Biosuspension führen, geleitet. Die halogenierten Ethene und/oder die halogenierten Butadiene permeieren durch die Membranen in die wässrige Biosuspension und werden dort abgebaut.

Soll der mikrobiologische Abbau der halogenierten Ethene und/oder der halogenierten Butadiene in den oben genannten Ausführungsformen unter sterilen Bedingungen erfolgen, so ist bei Verwendung von Blasensäulenreaktoren bzw. von Festbettreaktoren dem Reaktor ein Sterilfilter vorzuschalten, durch den der zu reinigende Gasstrom geleitet wird.

Um eine möglichst hohe spezifische Austauschfläche zu erhalten, wird bei einer weiteren Ausführungsform des erfindungsgemäßen Verfahrens in einer Emulsion gearbeitet. Dabei mischt man der wässrigen Phase, welche die präadaptierten Bakterien enthält, wegen des höheren Verteilungskoeffizienten als Adsorptionsmittel ein Öl zu, welches die abzubauenden halogenierten Ethene und/oder die halogenierten Butadiene bevorzugt aufnimmt. Solche Öle können z.B. Rapsöl sein oder auch Siliconöle. Die Verwendung von Rapsöl bietet den Vorteil, daß es sich bei Rapsöl um ein relativ billiges Öl handelt und daß dieses biologisch abbaubar ist. Zur Vergrößerung der Oberfläche zwischen der wässrigen und der lipophilen Phase und damit zur Beschleunigung des biologischen Abbaues der halogenierten und/oder der halogenierten Butadiene wird in einer weiter Ausführungsform des erfindungsgemäßen Verfahrens zur Stabilisierung der Emulsion ein Emulgator zugegeben. Hierbei kann es sich um biologische oder auch um synthetische Emulgatoren handeln. Geeignete biologische Emulgatoren sind z.b. Lecithine. Ein käuflich erwerbbares Produkt hierfür ist z.B. das Schneidöl Connoxol^{R} der Firma Leutze in Metzingen, welches aus Rapsöl und einem Emulgator besteht.

Da die Aktivität der präadaptierten Bakterien für den Abbau der halogenierten Ethene und/oder der halogenierten Butadiene wegen Nahrungsmangel im Laufe der Zeit nachläßt, muß bei einer kontinuierlichen Durchführung des erfindungsgemäßen Verfahrens entweder ständig neue, präadaptierte Bakterienmasse verwendet werden, oder bei nachlassender Aktivität der Bakterien müssen diese durch Zugabe von Isopren und/oder von Butadienen regeneriert und deren Enzymsystem neu induziert werden. Soll das erfindungsgemäße Verfahren kontinuierlich oder quasi kontinuierlich durchgeführt werden, so sind hierfür zwei Verfahrensvarianten möglich.

### Variante I :

Die präadaptierten Bakterien werden in einem Bioreaktor alternierend mit Isopren und/oder mit Butadienen für die Neubildung bzw. für die Regenerierung und die Induzierung und alternierend mit den abzubauenden halogenierten Ethenen und/oder den halogenierten Butadienen versorgt. Mit dieser Variante ist ein quasi kontinuierliches Verfahren ermöglicht, welches z.B. in einem Festbett-, in einem Blasensäulen- oder in einem Membranreaktor durchgeführt werden kann. Die Zeit, die zur Regenerierung der Bakterien und zur Induzierung des Enzymsystems erforderlich ist, hängt von den Erfordernissen des Einzelfalles ab. Werden z.B. Bakterien nach einer Abbauphase von Trichlorethylen mit Isopren regeneriert, und befinden sich diese Bakterien in einem Festbettreaktor, welcher mit Isopren gesättigt ist, so beträgt die Regenerierungszeit ca 10 - 20 % der Betriebszeit mit Trichlorethylen.

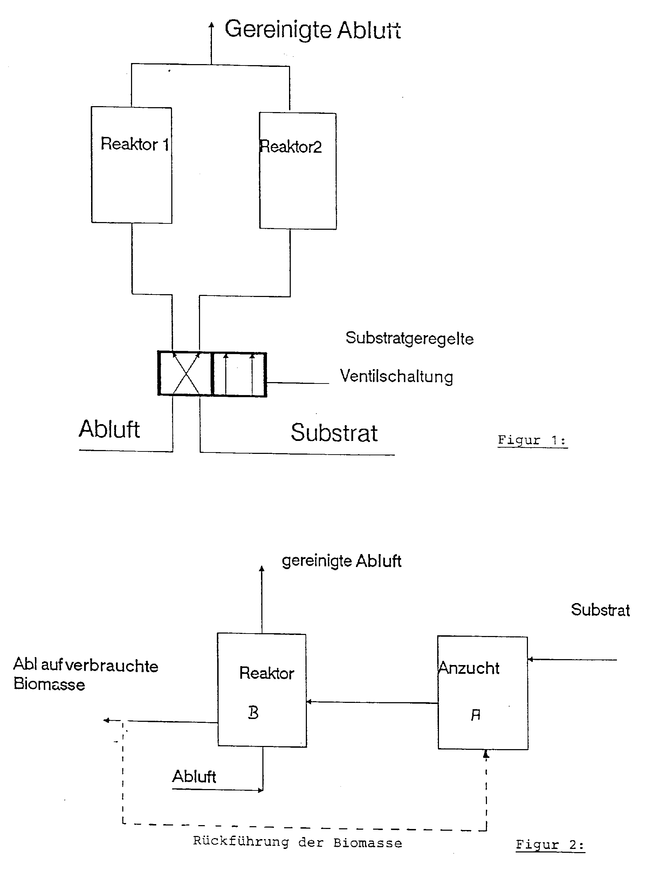

Figur 1 zeigt schematisch das erfindungsgemäße Verfahren gemäß der Variante I. Die Bioreaktoren (1) und (2) werden alternierend zum Abbau von z.B. Trichlorethylen eingesetzt. Der mit Trichlorethylen beladene Gasstrom wird in Reaktor (2) geleitet. Hier erfolgt der cooxidative Abbau des Trichlorethylen durch das induzierte Enzymsystem der präadaptierten Bakterien. Auf Grund des einsetzenden Mangels an NADH₂ sinkt die Abbaurate der Bakterien für Trichlorethylen. Vergleiche hierzu die Reaktionsgleichung von Seite 6 Zur Regenerierung der Bakterien und zur erneuten Induzierung des erforderlichen Enzymsystems wird der Gasstrom auf Reaktor (1) geschaltet, und der Reaktor (2) wird mit dem Wachstums- und Regenerierungssubstrat, z.B. mit Isopren, versorgt.

### Variante II :

Die Bakterien werden in einem Chemostaten präadaptiert und angezüchtet und dann in einen separaten Bioreaktor geleitet, in welchem der Abbau der halogenierten Ethene und/oder der halogenierten Butadiene stattfindet. Mit dieser Variante ist ein kontinuierliches Verfahren ermöglicht, welches z.B. in einem Blasensäulen- oder in einem Membranreaktor durchgeführt werden kann. In einer weiteren Ausführungsform des erfindungegemäßen Verfahrens wird die verbrauchte Biomasse in den Chemostaten zurückgeführt, dort mit Isopren und/oder mit Butadienen regeneriert und erneut in den Bioreaktor zum Abbau der halogenierten Ethene und/oder der halogenierten Butadiene zugeführt. Diese Verfahrensvariante ermöglicht bei einem Dauerbetrieb eine wesentlich ökonomischere Nutzung des Wachstums- bzw. Regenerierungssubstrates, da dieses nicht mehr als Kohlenstoffquelle für die Neubildung der Biomasse, sondern nur noch als Induktor für das erforderliche Enzymsystem und als Energiequelle zur Versorgung der Zellen mit NADH₂ benötigt wird.

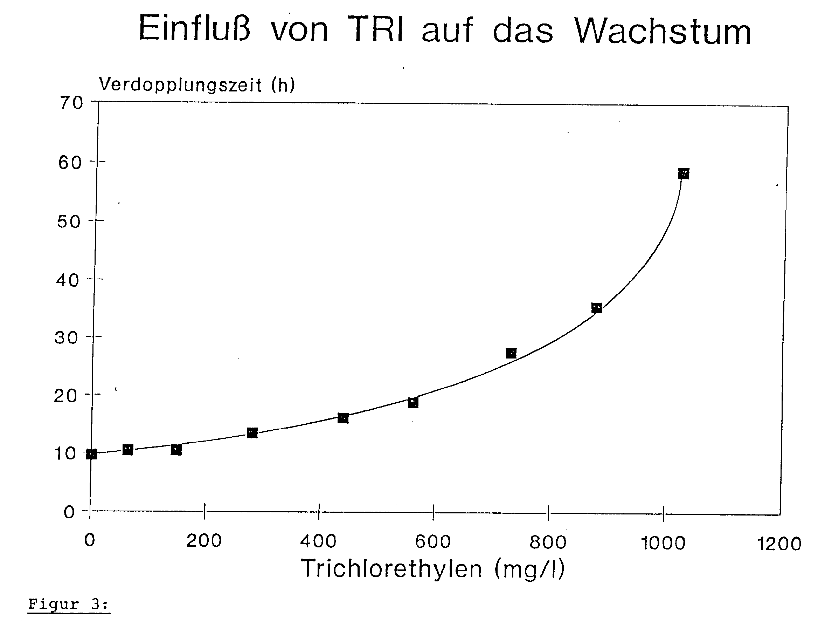

Figur 2 zeigt schematisch das erfindungsgemäße Verfahren gemäß der Variante II. Ein als Chemostat betriebener Reaktor (A) liefert ständig aktive, mit Isopren oder mit Butadien gezüchtete oder reaktivierte Biomasse. In einem nachgeschalteten Bioreaktor (B) erfolgt der cooxidative Abbau von z.B. Trichlorethylen. Die Verweilzeit der Zellen in diesem Bioreaktor wird so eingestellt, daß dort stets aktive Zellen zum Abbau des Trichlorethylens vorhanden sind. Läßt wegen Energiemangels die Abbauaktivität der Zellen nach, so können diese zur Regenerierung und zur Induzierung des erforderlichen Enzymsystems in den Chemostaten (A) zurückgeführt werden.

Die Wahl der Verfahrensvariante und des entsprechenden Bioreaktortyps hängt von den Erfordernissen des jeweiligen Anwendungsfalles ab. So wird man z.B. zur Durchführung des erfindungsgemäßen Verfahrens bei scherempfindliche Bakterien keinen Blasensäulenreaktor, sondern z.B. einen Festbettreaktor einsetzen.

Im Folgenden wird das erfindungsgemäße Verfahren anhand von Ausführungsbeispielen näher erläutert.

### Beispiel 1: (Gewinnung, Isolierung und Reinigung von geeigneten, präadaptierten Bakterien)

Alcaligenes denitrificans ssp. xylosoxidans JE 75 und Rhodococcus erythropolis JE 77 wurden mit Isopren (2-Methyl-1,3-butadien) als einziger Kohlenstoff- und Energiequelle aus einer Wasserprobe isoliert. Die Identifizierung der beiden Stämme erfolgte durch die Deutsche Sammlung für Mikroorganismen (DSM, Braunschweig). Für das Wachstum in Flüssigkultur wurde ein Mineralmedium verwendet, welches folgende Komponenten enthielt:
KH₂PO₄ : 3.4 g/l; Na₂HPO₄ x 2 H₂O : 4.4 g/l;
(NH₄)₂SO₄ : 1 g/l; Fe-(III)-NH₄-citrat : 10 mg/l;
MgSO₄ x 7 H₂O : 200 mg/l; Ca(NO₃)₂ x 4 H₂O : 50 mg/l;
Spurenelementlösung nach Pfennig und Lippert (N.Pfenning, K.D.Lippert, "Über das Vitamin B₁₂-Bedürfnis phototropher Schwefelbakterien", Arch.Mikrobiol. 55, S. 245 - 256 (1966)) Das Isopren wurde flüssig in die Kulturkolben (χᵢ=0.001) injiziert. Die Inkubation erfolgte in 100, 500 und 1000 ml Schikanenkolben mit jeweils 10, 50 oder 100 ml Mineralmedium bei 30 °C auf einem Schüttler (100 Upm). Das Wachstum wurde durch Trübungsmessung bei 546 nm in einem Beckmann-Phothometer (DU-50, Fullerton, California, USA) oder in einem Klett-Photometer bestimmt. Zur Reinigung und Isolierung wurden Agarplatten mit Agar No.1 (Oxoid Ltd., London, England) (ωᵢ = 1.5 %) und Mineralmedium hergestellt. Das Isopren wurde über die Gasphase (χᵢ = 10 %) zudosiert.

### Beispiel 2: (Screening in Mikrotiterplatten)

Die Abbauaktivität für chlorierte Ethene wurde über eine Bestimmung der Chloridmenge ermittelt. Der Chloridtest von Weightman et al (A.J.Weightman, A.L.Weightman, J.H.Slater, Appl. Environ. Microbiol. 49, S. 1494 - 1501 (1985)) wurde auf in Flüssigkultur angezogene Zellen übertragen. Die in 100 ml Schikanenkolben angezogenen Mischkulturen wurden zentrifugiert (6000 x g 15 min), zweimal mit 100 mM Tris-Sulfatpuffer (pH 8.0) gewaschen und auf eine optische Dichte von 15 - 20 eingestellt. Jeweils 100 µl Zellsuspension wurden mit 100 µl 100 mM Tris-Sulfatpuffer (pH 8.0), der 1 mM Trichlorethylen enthielt, versetzt und im Exsikkator bei 30 °C über Nacht inkubiert. Anschließend wurde jeder Testansatz mit 10 µl 0.1 M AgNO₃-Lösung versetzt und 15 min mit einer UV-Lampe (Desaga MinUVIS) bestrahlt. Das Auftreten von amorphen Silber ist ein Indikator für die Dehalogenierung, d.h. für den biologischen Abbau des Trichlorethylens.

Figur 3 zeigt die Toleranz von mit Isopren präadaptierten Rhodococcus erythropolis JE 77 gegenüber verschiedenen Trichlorethylen-Konzentrationen (TCE = Trichlorethylen). Die Anzucht erfolgte in 500 ml Klettphotometerkolben. Trichlorethylen wirde unverdünnt in den Kolben injiziert. Die TCE-Konzentrationen wurden unter Annahme vollständiger Löslichkeit in der wässrigen Phase kalkuliert. Ein Maß für die Toleranz der Bakterien ist deren Verdoppelungszeit. Diese wurde ermittelt, indem über Streulichtmessungen die optische Dichte der Bakteriensuspension in Abhängigkeit von der Zeit ermittelt wurde. Gezeigt wird die Hemmung des Wachstums auf Isopren durch unterschiedliche Trichlorethylen-Konzentrationen. Figur 3 zeigt, daß erst bei TCE-Konzentrationen ≧ 600 mg/l die Verdoppelungszeit drastisch steigt.

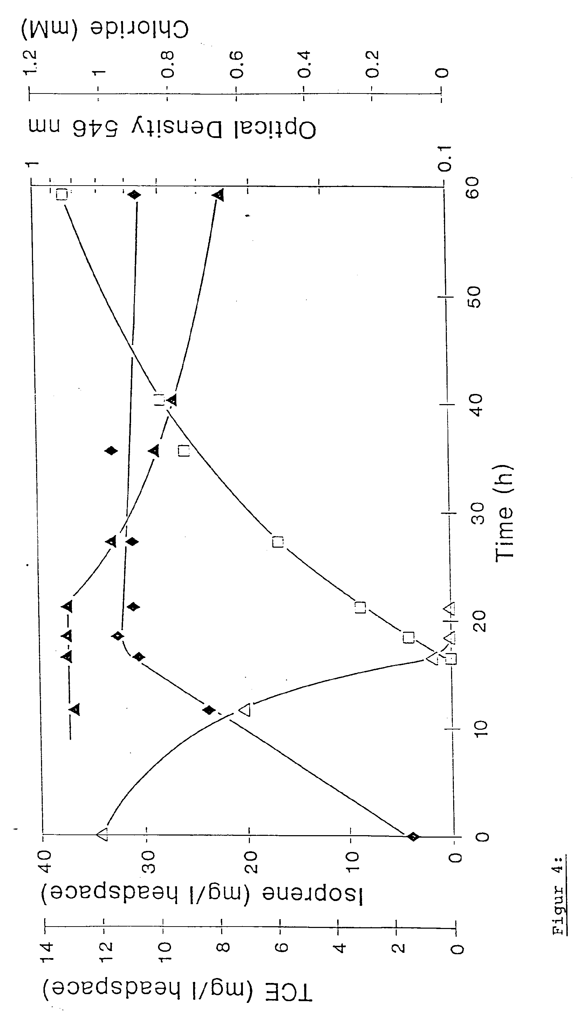

Selbst bei hohen TCE-Werten wurde noch eine Dehalogenierung, d.h. ein biologischer Abbau des Trichlorethylen beobachtet. Die Aktivität der Ruhezellen gegenüber Trichlorethylen bleibt mehrere Stunden erhalten und verlangsamt sich nur durch den allmählich auftretenden Energiemangel. Figur 4 zeigt das Wachstum von Rhodococcus erythropolis JE77 auf Isopren in Gegenwart von 15 ml Trichlorethylen. Die Anzucht erfolgte in einem 1000 ml Schikanenkolben, aus dem mit einer gasdichten Spritze Gasproben gezogen und auf Isopren (△) und auf Trichlorethylen (▲) untersucht wurden. (GC: Injektor-, Detektor- und Ofentemperatur waren 200 °, 300 ° und 200 °C, FID, Pora PLOT Q-Säule (25mx0.32) mit He (70 kPa Säulenvordruck) als Trägergas). Die Freisetzung von Chlorid (□) wurde mittels Ionenchromatographie bestimmt. Die optische Dichte (♢) wurde bei 546 nm gemessen. Die Dehalogenierung des Trichlorethylens fand während der stationären Phase statt und wurde über einen Zeitraum von 60 Stunden beobachtet.

## Patentansprüche

1. Verfahren zur mikrobiologischen Reinigung von mit halogenierten Ethenen und/oder mit halogenierten Butadienen kontaminierten Gasströmen, **dadurch gekennzeichnet,** daß man Bakterienstämme, welche mit Isopren und/oder mit Butadienen als Kohlenstoff- und Energiequelle wachsen und welche halogenierte Ethene und/oder halogenierte Butadiene abbauen, mit Isopren und/oder mit Butadienen als Kohlenstoff- und Energiequelle für die Neubildung oder für die Regenerierung präadaptiert und mit dem zu reinigenden Gasstrom in einem Bioreaktor kontaktiert.

2. Verfahren nach Anspruch 1, **dadurch gekennzeichnet,** daß man kontaminierte Gasströme reinigt, welche durch Einblasen von Luft in mit halogenierten Ethenen und/oder mit halogenierten Butadienen kontaminierten Böden oder Flüssigkeiten entstanden sind.

3. Verfahren nach Anspruch 1, **dadurch gekennzeichnet,** daß man kontaminierte Gasströme reinigt, welche durch Absaugen der Bodenluft aus mit halogenierten Ethenen und/oder mit halogenierten Butadienen kontaminierten Böden entstanden sind.

4. Verfahren nach einem der Ansprüche 1 bis 3, **dadurch gekennzeichnet,** daß man die Bakterien in einem Bioreaktor alternierend mit Isopren und/oder mit Butadienen versorgt und mit den zu reinigenden Gasstrom kontaktiert.

5. Verfahren nach einem der Ansprüche 1 bis 3, **dadurch gekennzeichnet,** daß man die Bakterien in einem Chemostaten anzüchtet und präadaptiert, und in einem separaten Bioreaktor mit den zu reinigenden Gasstrom kontaktiert.

6. Verfahren nach Anspruch 5, **dadurch gekennzeichnet,** daß man die Bakterien nach dem Kontaktieren mit dem zu reinigenden Gasstrom zurück in den Chemostaten führt.

7. Verfahren nach einem oder mehreren der Ansprüche 1 bis 6, **dadurch gekennzeichnet,** daß man die Bakterien mit Isopren und/oder mit Butadienen über die Gasphase versorgt und gasförmiges Isopren und/oder gasförmige Butadiene dem Luftstrom für die Begasung des Bioreaktors zumischt.

8. Verfahren nach einem oder mehreren der Ansprüche 1 bis 7, **dadurch gekennzeichnet,** daß man die präadaptierten Bakterien in einer Emulsion aus einer die Bakterien enthaltenden, wässrigen Phase und einem die halogenierten Ethene und/oder die halogenierten Butadiene aufnehmenden Öl mit dem zu reinigenden Gasstrom kontaktiert.

9. Verfahren nach einem oder mehreren der Ansprüche 1 bis 7, **dadurch gekennzeichnet,** daß man den zu reinigenden Gasstrom in einen Festbettreaktor über die an Füllkörpern immobilisierten, präadaptierten Bakterien leitet.

10. Verfahren nach einem oder mehreren der Ansprüche 1 bis 7, **dadurch gekennzeichnet,** daß man den zu reinigenden Gasstrom in einen Blasensäulenreaktor durch die wässrige Biosuspension der präadaptierten Bakterien leitet.

11. Verfahren nach einem oder mehreren der Ansprüche 1 bis 7, **dadurch gekennzeichnet,** daß man den zu reinigenden Gasstrom in einen Membranreaktor durch Hohlfasermembranen leitet, durch die die abzubauenden Schadstoffe in die wässrige, die präadaptierten Bakterien enthaltende Biosuspension permeieren.

12. Verfahren nach einem oder mehreren der Ansprüche 1 bis 11, **dadurch gekennzeichnet,** daß man als Kohlenstoffquelle für die Neubildung und/oder für die Regenerierung der Bakterien bevorzugt Isopren oder 1,3-Butadien verwendet.

13. Verfahren nach einem oder mehreren der Ansprüche 1 bis 12, **dadurch gekennzeichnet,** daß man bevorzugt Gasströme reinigt, die chlorierte Ethene enthalten.

14. Verfahren nach Anspruch 13, **dadurch gekennzeichnet,** daß man Gasströme mit Konzentrationen an chlorierten Ethenen von weniger als 100 mg/m³ reinigt.

15. Verfahren nach einem oder mehreren der Ansprüche 1 bis 14, **dadurch gekennzeichnet,** daß man Bakterien präadaptiert, die erhalten worden sind, indem man aus Boden- oder Wasserproben mittels Isopren und/oder mittels Butadienen als einziger Kohlenstoff- und Energiequelle Isopren und/oder Butadien abbauende Bakterien isoliert und mit Hilfe eines Schnelltestes nach bekannten Methoden aus diesen halogenierte Ethene und/oder halogenierte Butadiene abbauende Bakterien ermittelt und isoliert.

16. Verfahren nach einem oder mehreren der Ansprüche 1 bis 15, **dadurch gekennzeichnet,** daß man Bakterienstämme der Gattung Alcaligenes denitrificans ssp. xylosoxidans oder Rhodococcus erythropolis präadaptiert.

## Claims

1. Method of microbiologically purifying gas currents contaminated with halogenated ethenes and/or halogenated butadienes, **characterized in** that bacterial strains which grow with isoprene and/or butadienes as carbon and energy source and which decompose halogenated ethenes and/or halogenated butadienes are pre-adapted with isoprene and/or with butadienes as carbon and energy source for the new formation or the regeneration and are contacted with the gas current to be purified in a bioreactor.

2. Method according to Claim 1, **characterized in** that contaminated gas currents are purified which have been created by blowing air into soils or liquids contaminated with halogenated ethenes and/or halogenated butadienes.

3. Method according to Claim 1, **characterized in** that contaminated gas currents are purified which have been created by exhausting air from soils contaminated with halogenated ethenes and/or halogenated butadienes.

4. Method according to any of Claims 1 to 3, **characterized in** that in a bioreactor said bacteria are supplied in alternation with isoprene and/or butadienes, and are contacted with the gas current to be purified.

5. Method according to any of Claims 1 to 3, **characterized in** that said bacteria are grown and pre-adapted in a chemostatic system, and are contacted with the gas current to be purified in a separate bioreactor.

6. Method according to Claim 5, **characterized in** that said bacteria having been contacted with the gas current to be purified are returned into said chemostatic system.

7. Method according to one or several of Claims 1 to 6, **characterized in** that said bacteria are supplied with isoprene and/or with butadienes through the vapor phase, and that gaseous isoprene and/or gaseous butadienes are admixed to the air flow for gassing said bioreactor.

8. Method according to one or several of Claims 1 to 7, **characterized in** that said pre-adapted bacteria are contacted with the gas current to be purified in an emulsion of an aqueous phase containing said bacteria and an oil absorbing said halogenated ethenes and/or said halogenated butadienes.

9. Method according to one or several of Claims 1 to 7, **characterized in** that the gas current to be purified is passed into a fixed-bed reactor over said pre-adapted bacteria immobilized on packing elements.

10. Method according to one or several of Claims 1 to 7, **characterized in** that the gas current to be purified is passed into a bubble-cap column reactor through the aqueous bio-suspension of said pre-adapted bacteria.

11. Method according to one or several of Claims 1 to 7, **charactetized in** that the gas current to be purified is passed into a membrane reactor through hollow-fibre membranes which the pollutants to be decomposed permeate through into said aqueous bio-suspension containing said pre-adapted bacteria.

12. Method according to one or several of Claims 1 to 11, **characterized in** that preferably isoprene or 1,3-butadiene is employed as carbon source for the new formation and/or regeneration of said bacteria.

13. Method according to one or several of Claims 1 to 12, **characterized in** that preferably gas currents are purified which contain chlorinated ethenes.

14. Method according to Claim 13, **characterized in** that gas currents are purified which present concentrations of chlorinated ethenes of less than 100 mg/m³.

15. Method according to one or several of Claims 1 to 14, **characterized in** that bacteria are pre-adapted which had been obtained by isolating bacteria decomposing isoprene and/or butadiene from soil or water specimens by means of isoprene and/ or by means of butadienes as the only source of carbon and energy, and that bacteria decomposing halogenated ethenes and/ or halogenated butadienes are determined and isolated by means of a quick test according to methods known per se.

16. Method according to one or several of Claims 1 to 15, **characterized in** that bacterial strains of the variety *Alcaligenes* *denitrificans ssp*. *xylosoxidans* or *Rhodococcus erythropolis* are pre-adapted.

## Revendications

1. Procédé d'épuration microbiologique de courants de gaz contaminés par des éthylènes halogénés et/ou des butadiènes halogénés, **caractérisé en ce** que des sous-règnes de bactéria qui croissent en combinaison avec l'isoprène et/ou les butadiènes comme source de carbone et d'énergie et qui décomposent des éthylènes halogénés et/ou des butadiènes halogénés sont pré-adaptés en combinaison avec l'isoprène et/ou les butadiènes comme source de carbone et d'énergie pour la nouvelle formation ou la régénération, et sont contactés avec le courant de gaz à épurer dans un bioréacteur.

2. Procédé selon la revendication 1, **caractérisé en ce** que des courants de gaz contaminés sont épurés, qui sont produits en soufflant de l'air dans des sols ou des liquides contaminés par des éthylènes halogénés et/ou des butadiènes halogénés.

3. Procédé selon la revendication 1, **caractérisé en ce** que des courants de gaz contaminés sont épurés, qui sont produits en aspirant de l'air des sols contaminés par des éthylènes halogénés et/ou des butadiènes halogénés.

4. Procédé selon une quelconque des revendications 1 à 3, **caractérisé en ce** que, dans un bioréacteur, lesdits bacteria sont alimentés, à tour de rôle, en isoprène et/ou les butadiènes, et sont contactés avec le courant de gaz à épurer.

5. Procédé selon une quelconque des revendications 1 à 3, **caractérisé en ce** que lesdits bactéria sont cultivés et pré-adaptés dans un système chimiostatique, et sont contactés, dans un bioréacteur séparé, avec le courant de gaz à épurer. .

6. Procédé selon la revendication 5, **caractérisé en ce** que lesdits bactéria, après leur contact avec le courant de gaz à épurer, sont retournés dans ledit système chimiostatique.

7. Procédé selon une ou plusieurs des revendications 1 à 6, **caractérisé en ce** que lesdits bactéria sont alimentés en isoprène et/ou les butadiènes par la phase gazeuse, et en ce que ledit isoprène gazeux et/ou les butadiènes gazeux sont mélangé avec le courant d'air pour l'introduction de gaz dans ledit bioréacteur.

8. Procédé selon une ou plusieurs des revendications 1 à 7, **caractérisé en ce** que lesdits bactéria pré-adaptés sont contactés avec le courant de gaz à épurer dans une émulsion d'une phase aqueuse qui contient lesdits bactéria et une huile qui absorbe lesdits éthylènes halogénés et/ou lesdits butadiènes halogénés.

9. Procédé selon une ou plusieurs des revendications 1 à 7, **caractérisé en ce** que le courant de gaz à épurer est passé dans un réacteur à lit fixe en s'écoulant sur lesdits bactéria pré-adaptés et immobilisés sur des corps de remplissage.

10. Procédé selon une ou plusieurs des revendications 1 à 7, **caractérisé en ce** que le courant de gaz à épurer est passé dans un réacteur à colonne à bulles par ladite bio-suspension aqueuse desdits bactéria pré-adaptés.

11. Procédé selon une ou plusieurs des revendications 1 à 7, **caractérisé en ce** que le courant de gaz à épurer est passé dans un réacteur à membrane par des membranes à fibres creuses qui sont traversées par les substances nocives à décomposer, dans ladite bio-suspension qui contient lesdits bactéria pré-adaptés.

12. Procédé selon une ou plusieurs des revendications 1 à 11, **caractérisé en ce** que, de préférence, l'isoprène ou le 1,3-butadiène est utilisé comme source de carbone pour la nouvelle formation et/ou la régénération desdits bactéria.

13. Procédé selon une ou plusieurs des revendications 1 à 12, **caracterisé en ce** que, de préférence, des courants de gaz sont épurés qui contiennent des éthylènes chlorés.

14. Procédé selon la revendication 13, **caractérisé en ce** que des courants de gaz sont épurés qui présentent des concentrations des éthylènes chlorés à moins de 100 mg/m³.

15. Procédé selon une ou plusieurs des revendications 1 à 14, **caractérisé en ce** que des bactéria sont pré-adaptés, qu'on avait obtenu en isolant des bactéria capable de décomposer l'isoprène et/ou le butadiène, des spécimens de sol ou de l'eau moyennant l'isoprène et/ou moyennant des butadiènes comme la seule source de carbone et d'énergie, et en ce que des bactéria capable de décomposer des éthylènes halogénés et/ou des butadiènes halogénés sont détectés et isolés par un essai accéléré selon des procédés connus en soi.

16. Procédé selon une ou plusieurs des revendications 1 à 15, **caractérisé en ce** que des sous-règnes de bactéria de l'espèce *Alcaligenes denitrificans ssp. xylosoxidans* ou *Rhodococcus ery**thropolis* sont pré-adaptés.
